(19)

Europäisches Patentamt
European Patent Office
Office européen des brevets

(11) **EP 2 423 675 A2**

(12) **EUROPEAN PATENT APPLICATION**

(43) Date of publication:
**29.02.2012 Bulletin 2012/09**

(51) Int Cl.:
*G01N 22/00* [(2006.01)]  *G01N 22/04* [(2006.01)]
*G01N 33/02* [(2006.01)]  *G01N 33/06* [(2006.01)]

(21) Application number: **11178938.4**

(22) Date of filing: **25.08.2011**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **31.08.2010 US 873067**

(71) Applicant: **General Electric Company
Schenectady, NY 12345 (US)**

(72) Inventors:
• **Webster, Jack Mathew
  Niskayuna, NY 12309 (US)**
• **Neculaes, Vasile Bogdan
  Niskayuna, NY 12309 (US)**

(74) Representative: **Illingworth-Law, William
Illingworth
Global Patent Operation - Europe
GE International Inc.
15 John Adam Street
London WC2N 6LU (GB)**

(54) **System and method for measuring calorie content of a food sample**

(57)      A system includes an estimating unit (21) to non-destructively estimate a fat content and a water content of a food sample (S). The system further includes a processing unit (29) operatively coupled to the estimating unit (21) to determine a calorie content based solely on the fat content and the water content of the food sample (S). The estimating unit (21) may include a microwave spectrometer (70) with a transmitter (22a) and a receiver (22b). The transmitter (22a) can be capable of transmitting microwaves (W) of multiple frequencies through a food sample (S). The received signal can then be compared to the transmitted signal to estimate the fat content and water content.

*Fig. 2*

EP 2 423 675 A2

**Description**

BACKGROUND

Embodiments presented herein are directed generally to measuring a calorie content of a food sample, and more specifically to measuring the calorie content of the food sample non-destructively.

In order to effectively control one's weight, it is necessary to provide a proper balance between the caloric input and the number of calories burned. Whether a user is following a specific diet, a particular exercise regimen, is on weight gain/loss program or had a gastric bypass surgery, one has to correlate calorie consumption with the number of calories burned. Even if the user wishes to merely maintain his weight, it is necessary to balance the number of calories consumed and the number of calories burned, as in this case both should be approximately same.

The calories are burned as a result of specific exercises/physical activities done by the user. In calculating the number of calories burned, the user must take into consideration the type of activity in which he is engaged. The number of calories burned is a function of the level of activity and also dependent upon the particular characteristics of the individual, such as the weight, age and sex. The users are accustomed to automated monitoring of calories burned. Most modem exercise machines display an estimate of the number of calories burned. Further, the users wear accelerometer based activity monitors to automatically translate daily body movements to calories burned.

On the other hand, in recording the number of calories consumed, the user must have some information readily available which indicates the number of calories per unit quantity of various food items he is consuming. Keeping track of calories consumed remains a fairly manual and time-consuming task. It requires the user to measure the weight or volume of each food item eaten and to find the calories of that particular food item from an index (either a book or online). One has to then translate the index units to the amount of food eaten and record in a diet journal.

Further, many of the food items eaten are not accurately described by a value in the index and are variable in their calorie densities. The calorie content of the food items consumed varies widely depending on the ingredients and amounts of those ingredients. One way around this problem is to manually index each ingredient in a recipe and add them up; but this requires even more effort. The actual calorie content of a meal can vary widely depending upon the actual quantities of ingredients used in the preparation of the meal.

There is therefore a need for a system that allows the users to get an empirical estimate of the calorie content of the food items they are consuming. There is a further need for a system and method that estimate the calorie content of the food items non-destructively.

BRIEF DESCRIPTION

Briefly, in accordance with aspects of the present technique, a system including an estimating unit and a processing unit to non-destructively estimate a fat content and a water content of a food sample is presented. The processing unit is operatively coupled to the estimating unit to determine a calorie density based solely on the estimated fat content and the water content of the food sample.

In accordance with another aspect of the present technique, a method of estimating a fat content and a water content of a food sample is presented. The fat content and the water content are estimated with an estimating unit. The method further includes determining a calorie density of the food sample based solely on the estimated fat content and the water content using a processing unit. The processing unit is operatively coupled to the estimating unit.

In accordance with further aspects of the present technique, a method of estimating a fat content and a water content of a food sample is presented. The method includes transmitting microwave radiation such that at least a part of the microwave radiation interacts with a food sample. The method further includes receiving at least some of the transmitted microwave radiation. The method also includes estimating a fat content and a water content of the food sample based on the received microwave radiation. The method includes determining a calorie density of the food sample based solely on the estimated fat content and the water content.

DRAWINGS

These and other features, aspects, and advantages of the present invention will become better understood when the following detailed description is read with reference to the accompanying drawings in which like characters represent like parts throughout the drawings, wherein:

FIG. 1 is a diagrammatical illustration of a system for communicating weight, calories consumed and calories burned to a user, in accordance with aspects of the present technique;

FIG. 2 is a diagrammatical illustration of a system for measuring calorie content of a food sample, in accordance with aspects of the present technique;

FIG. 3 is a flowchart illustrating a method of determining calorie content of a food sample, in accordance with aspects of the present technique;

FIG. 4 is a flowchart illustrating a method of estimating fat content and water content of a food sample, in accordance with aspects of the present technique;

FIG. 5 is a flowchart illustrating a detailed method of determining calorie content of a food sample, in accordance with aspects of the present technique;

FIG. 6 is a flowchart illustrating a method of generating a regression expression, in accordance with aspects of the present technique;

FIG. 7 is a plot of water content vs. calorie density of fat-free food items reported in a data repository, in accordance with aspects of the present technique;

FIG. 8 is a plot of water content vs. calorie density of fat-containing food items reported in the data repository, in accordance with aspects of the present technique;

FIG. 9 is a plot of fat content vs. Δ calorie density, in accordance with aspects of the present technique; and

FIG. 10 is a plot of empirical calorie density obtained from the data repository vs. calorie density predicted from a third equation using fat content and water content obtained from the data repository, in accordance with aspects of the present technique.

DETAILED DESCRIPTION

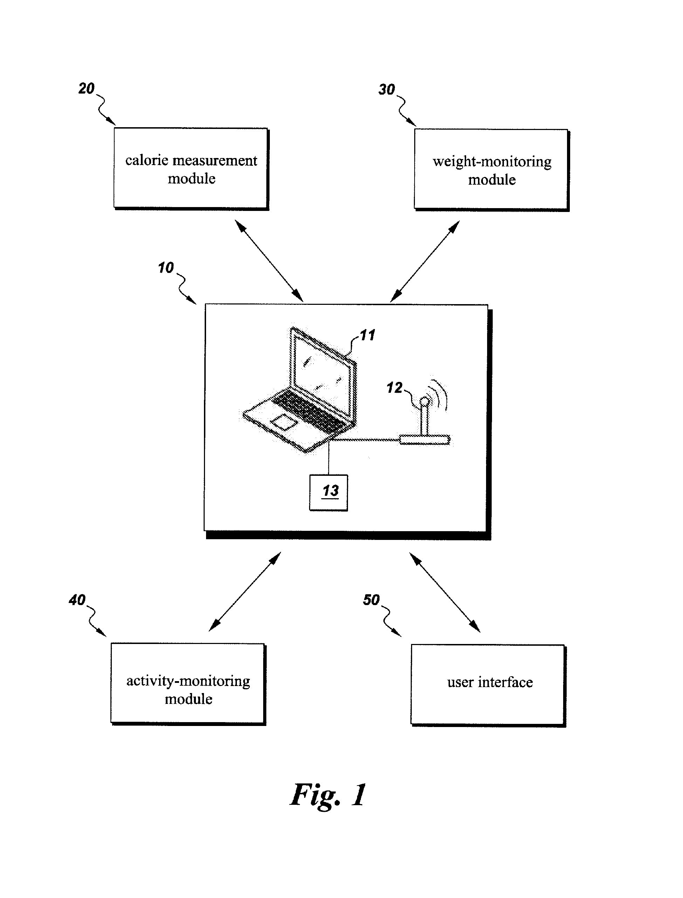

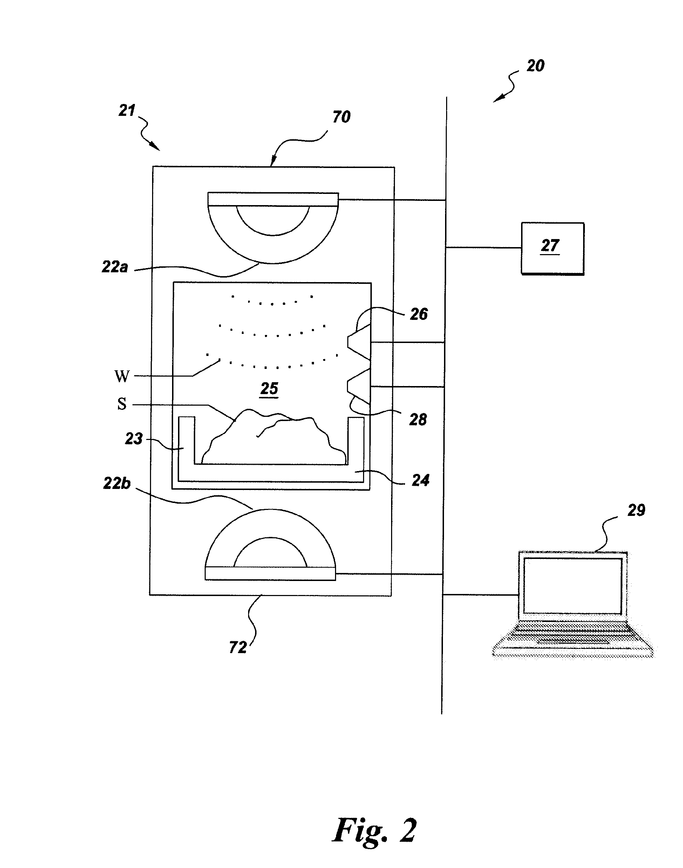

Referring to FIGS. 1 and 2, therein is illustrated an example system 100 including a health management module 10. The health management module 10 can include a computing device, such as, for example, a computer 11, a smartphone, and/or the like, which may be configured to execute a health management application. The computer 11 may include hardware that is configured to execute the health management application, such as an application-specific integrated circuit, or may include or receive (say, via the Internet) instructions (*e.g.*, software) to be executed by a general-purpose central processing unit of the computer. In any event, the health management application, when executed by the computer 11, may present a graphical user interface that enables a user to track his or her weight, the calories consumed, and the calories burned in real time. The health management module 10 may communicate with a storage device 13, such as, for example, a random access memory (RAM), which storage device may be included in the computer 11 or may be located remotely and accessible, say, via a local network and/or the Internet. In one embodiment, software associated with the health management application may be stored in the storage device 13. The health management module 10 may include a wireless transmitter/receiver 12 that can facilitate uploading data from various external sources to the health management module.

The system 100 may further include a calorie measurement module 20. The calorie measurement module 20 can include an estimating unit 21 that can be configured to collect data that, as discussed below, represents (and enables subsequent estimation of) the fat content and the water content of a food sample S that is disposed in the estimating unit. A computing device 29, which, in one embodiment, may be one or more of a computer, smartphone, and/or the like, can be coupled to the estimating unit 21. Data collected by the estimating unit 21 can be transmitted to the computing device 29 for subsequent use in estimation of the fat content and the water content of the food sample S and calculation of the calorie content of the food sample. The calorie measurement module 20 may also include a memory 27 (*e.g.*, RAM) that is operatively coupled to the estimating unit 21 and the computing device 29, which memory may store data collected by the estimating unit and/or data processed or to be processed by the computing device. In one example embodiment, the estimating unit 21 can include a spectrometer (for example, a microwave spectrometer, a near infrared spectrometer, an ultra-wide band pulse dispersion microwave spectrometer, and/or the like). A process by which data representing the fat content and the water content of a food sample can be used to estimate the fat content and water content of the sample and calculate the calorie content of the sample is described below in further detail.

The system 100 further may also include a weight-monitoring module 30 and an activity-monitoring module 40. The weight-monitoring module 30 may include a simple weighing scale to measure the weight of the user, and/or may include a machine configured to measure the Body Mass Index (BMI). The activity-monitoring module 40 may include an automated monitor to track the calories burned by the user. In one embodiment, the activity-monitoring module 40 may include a wearable device, such as, for example, a pedometer, a three-dimensional accelerometer, a heart rate monitor, and/or the like. The activity-monitoring module 40 may be suitably calibrated so as to convert measurements of activity into calories burned.

The health management module 10 may be operatively coupled to the calorie measurement module 20, the weight-monitoring module 30, and/or the activity-monitoring module 40, say, via the wireless transmitter 12. The calorie measurement module 20, the weight-monitoring module 30, and/or the activity-monitoring module 40 may therefore transmit data collected thereby to the health management module 10, for example, so as to be stored by the storage device 13. Aside from weight and calorie consumption data, the storage device 13 may also retain historical health data.

A user interface 50 may be communicatively coupled to the health management module 10 and may provide an indication of weight/BMI information obtained from the weight monitoring module 30, calorie content obtained from the calorie measurement module 20, and the burned calories obtained from the activity monitoring module 40. The user interface 50 may be, for example, a wearable device or an electronic card that allows the user to view the calories consumed and the calories burned throughout the day. It should be further noted that the user interface 50 and the activity-monitoring module 40 may exist as an application running on a single wireless device, such as a cellular telephone, a portable computing device (*e.g.*, a smartphone, a laptop computer, or an application-specific device), etc., which computing device may coincide with the computing device 11 of the health management module 10.

In some embodiments, the system 100 may exclude the weight-monitoring module 30, the activity-monitoring module 40, and/or the user interface 50. The system 100 may instead be configured such that the user can enter weight and exercise information directly into the system 100, say, via the health management module 10. Alternatively, the system 100 may be configured such that a user may enter weight and exercise information into, for example, the user interface 50.

A food sample for which the fat content and the water content are to be estimated and the calorie content calculated can be placed in the estimating unit 21. The calorie measurement module 20 can then estimate the fat content and the water content of the food sample and calculate the calorie content. Specifically, the estimating unit 21 can collect data that enable estimation of the fat content and the water content of the sample. The processing unit 29 may then determine a calorie content of the food sample, for example, based solely on the estimated fat and water content. The information on the calorie content of the food sample can be uploaded via the wireless transmitter 12 to the health management module 10. The operation of the calorie measurement module 20 will be described in detail below with reference to FIGS. 2-4.

Referring to FIG. 2, the estimating unit 21 may include a microwave spectrometer 70. The microwave spectrometer 70 may include a transmitter 22a and a receiver 22b. The transmitter 22a can be, for example, a low power microwave transmitter capable of transmitting microwaves of multiple frequencies throughout a free space region 25 of the microwave spectrometer 70 (*e.g.*, where a food sample S can be placed). The estimating unit 21 may also include a weighing scale 24 and a holding unit 23 that retains the food sample S within the spectrometer 70. In one embodiment, the weighing scale 24 may be integrated into the microwave spectrometer 70 (say, into a housing 72 of the spectrometer). An optical scanner 26, such as, for example, a low-resolution, three-dimensional optical scanner, may also be included. A temperature measuring device, such as, for example, a thermometer, a thermocouple, or an infrared thermometer 28, may be included as well. Each of the transmitter 22a, receiver 22b, scale 24, optical scanner 26, and infrared thermometer 28 may be operatively connected to the processing unit 29.

In operation, the transmitter 22a may selectively transmit microwaves W into the free space region 25 of the microwave spectrometer 70. For example, the calorie measurement module 20 may be configured to allow a user to enter a command (say, by pressing a button) that results in a signal being sent by the processing unit 29 to the spectrometer 70 to initiate the transmission of microwaves from the transmitter 22a. A portion of the transmitted microwaves *W* can interact with the food sample S, and the receiver 22b can subsequently receive the propagating microwaves.

The propagating waves *W* have associated therewith various wave parameters, including, for example, amplitude, phase, attenuation, cut-off frequency, and phase shift. For microwaves propagating through the free space region (*i.e.*, without interacting with a food sample), these parameters can be determined as a function of the geometry of the spectrometer 70 and the properties of the transmitter 22a, and can be stored, say, in the memory 27. As the emitted microwaves W travel from the transmitter 22a to the receiver 22b and interact with the food sample S, the wave parameters of the propagating microwaves will be perturbed due to the presence of the food sample. For example, as the microwaves *W* interact with the food sample, polar molecules disposed in the water and fats in the food sample may rotate so as to align with the electromagnetic field associated with the propagating wave, this rotation affecting the properties of the wave itself. Changes in the parameters associated with the waves W due to interactions with the food sample S can therefore provide information about the food sample.

The wave data collected by the receiver 22b can be communicated to the processing unit 29 to extract therefrom the wave parameter data for the received waves. The received wave parameter data can then be compared to the wave parameter data for the waves initially transmitted from the transmitter 22a to determine the magnitude of the perturbation of the wave parameters due to the interaction of the waves *W* with the food sample *S*, and, as discussed in more detail below, thereby estimate the fat content (mass of fat/total mass of food sample) and water content (mass of water/total mass of food sample). It is noted that the above-described process for estimating fat and water content does not require destruction of the measured food sample. For more information concerning the relationship between wave parameter perturbations and determinations therefrom of fat content and water content, see Buford Randall Jean, "Process Composition Monitoring at Microwave Frequencies: A Waveguide Cutoff Method and Calibration Procedure," IEEE Transactions on Instrumentation and Measurement, Vol. 55(1), February 2006; U.S. Pat. No. 7,221,169 to Jean et al.*,* and U.S. Pat. No. 5,331,284 to Jean et al.*,* the content of each being incorporated herein by reference in its entirety.

It is noted that the microwaves W travelling from the transmitter 22a to the receiver 22b may be somewhat affected by various system variables, including, for example, the total mass, volume, density, geometry, and temperature of the food sample being measured. The extent to which these variables may affect the propagating microwaves can depend, for example, on the uniformity of the electromagnetic field associated with the propagating microwaves. The microwave spectrometer 70 can be provided with a scale 24 that can be used to measure the mass of the food sample, an optical scanner 26 that measures the volume of the food sample $S$, and an infrared thermometer 28 that measures the temperature of the food sample. The processing unit 29 of the microwave spectrometer 70 may then be configured to calibrate readings of the estimated fat and water content for varying total mass, volume, density, and temperature of the food sample. For example, measurements of food samples with known compositions can be repeated several times while independently varying total mass, volume, density, geometry, and temperature, thereby quantifying the effect of each variable. As will be appreciated by those skilled in the art, in this way, the microwave spectrometer 70 can be calibrated to estimate the fat content and the water content of a food sample with arbitrary total mass, volume, density, and temperature.

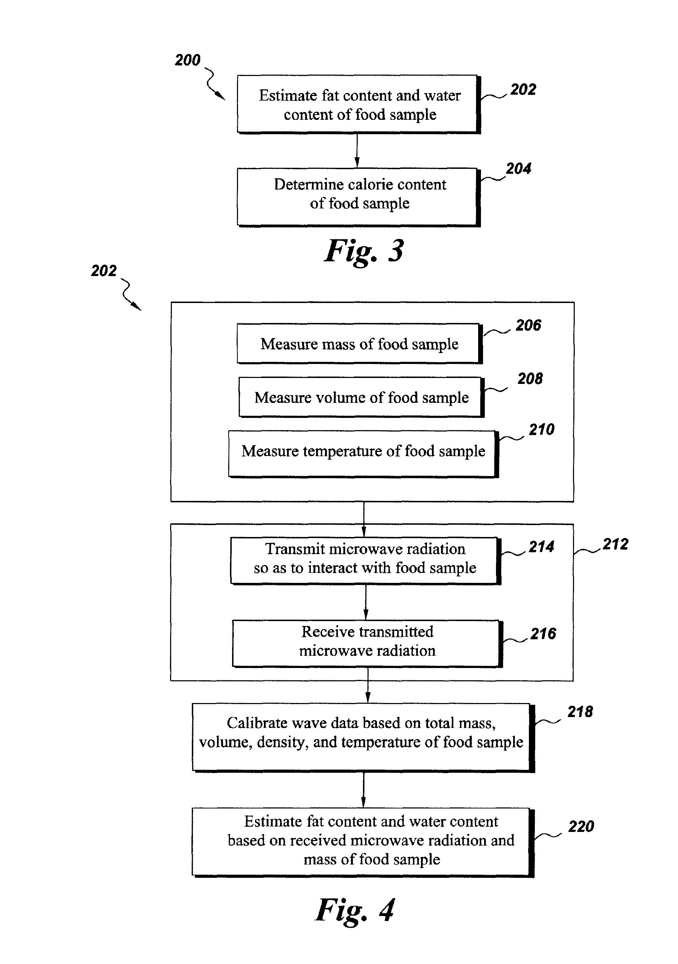

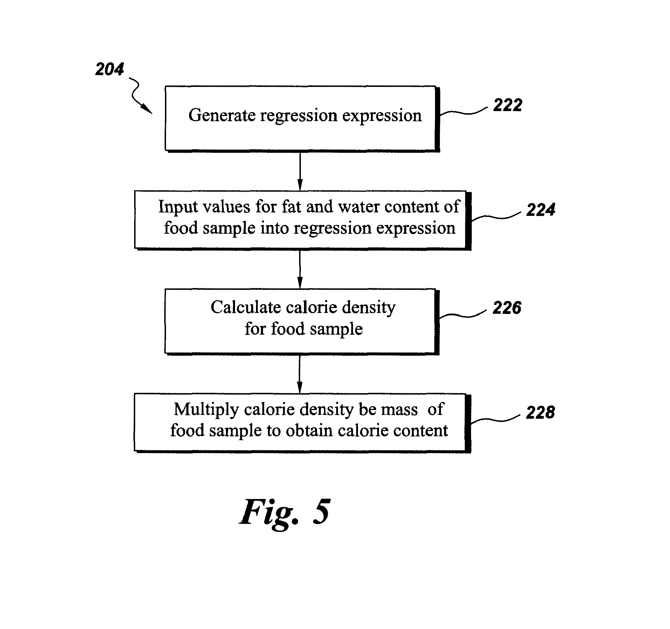

FIG. 3 is a flow chart of an example method 200 for determining the calorie content of a food sample using a system consistent with the system 100 depicted in FIG. 1. Referring to FIGS. 1-3, the method 200 can include estimating (202) a fat content and a water content of a food sample and determining (204) a calorie content of the food sample. The fat content and the water content of the food sample can be estimated, for example, using the estimating unit 21. The estimating (202) of fat content and water content is explained in greater detail below in conjunction with FIG. 4. The calorie content of the food sample can be determined, say, by the processing unit 29 using the estimated fat content and water content of the food sample to determine the calorie density of the food sample. The determination (204) of calorie content will be explained in detail below in conjunction with FIG. 5.

Referring to FIGS. 2 and 4, the estimation (202) of the fat content and the water content through the use of the estimating unit 21 is represented in detail in FIG. 4. The mass of the food sample S can be measured (206), for example, using the weighing scale 24. The volume of the food sample $S$ can be measured (208) using, for example, the optical scanner 26. The temperature of the food sample S can be measured (210), for example, using the infrared thermometer 28. The food sample can be probed (212) using electromagnetic radiation. More specifically, microwaves W can be emitted (214), say, by the transmitter 22a (*e.g.*, in response to a signal from the processing unit 29) and received (216) by the receiver 22b.

The fat content and the water content of the food sample $S$ can then be estimated (220), for example, by the processing unit 29 after receiving wave data from the transmitter 22a and receiver 22b. For example, as mentioned above, wave parameters can be extracted or otherwise determined for the transmitted and received microwaves $W$, and differences in the transmitted wave parameters and received wave parameters can be analyzed to determine fat and water content of the food sample $S$. Prior to estimating (220) the fat and water content of the food sample $S$, the wave data can be calibrated (218), if needed, for total mass, volume, density, and/or temperature of the food sample.

FIG. 5 is a flow chart describing in detail the determination (204, FIG. 3) of calorie content of a food sample using a system consistent with the system 100 depicted in FIG. 1. Referring to FIGS. 1, 2, and 5, a regression expression that relates fat and water content to calorie density can be generated (222). In some embodiments, the generation of the regression expression can be accomplished by the processing unit 29, while in other cases the regression expression may be generated separately and stored, say, in the memory 27. Further details regarding the form of the regression expression are provided below. Values for the estimated fat content and water content of a food sample S can be inputted (224) into the generated expression (say, by the processing unit 29), and the calorie density $CD$ (calories/unit mass) of the food sample can thereby be calculated (226). The mass of the food sample $S$, having been determined earlier, say, by the scale 24, can be multiplied (228) by the calorie density $CD$ (again, *e.g.,* by the processing unit 29) to obtain the calorie content of the food sample.

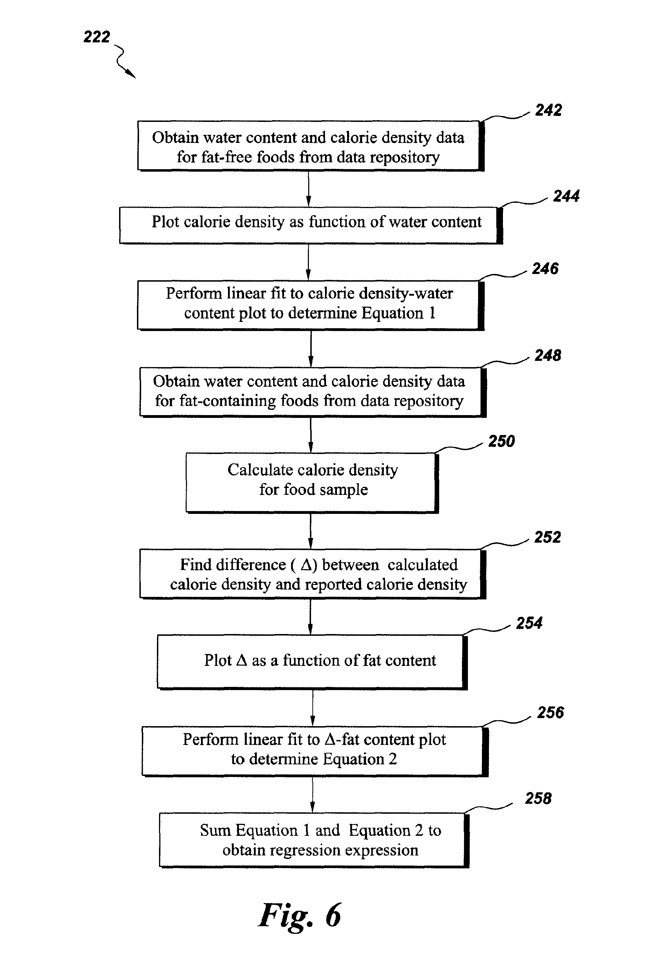

Though the method 204 is depicted in FIG. 5 as beginning with the generation (222) of a regression expression, it should be appreciated that a regression expression, once generated, may be stored in the memory 27 of the calorie measurement module 20. As such, subsequent uses of method 204 may begin with the previously generated regression expression simply being retrieved. The detailed procedure for generating (222) the regression expression will be described in detail below in conjunction with FIG. 6.

The method 200 (FIG. 3), when utilized in conjunction with the calorie measurement module 20, can allow the user to place a food sample S in the estimating unit 21 and, say, press a button to initiate a measurement of the calorie content of the food sample. The food sample may be a representative sample of a larger food item or a batch of food items. This method can thus enable the estimation of calorie content of the larger food item or the batch of food items just by measuring the fat and water content the food sample. Further, because calorie content is measured non-destructively, this method may further enable the user to place a meal containing arbitrary food items in the estimating unit 20 and get the calorie content of the entire meal.

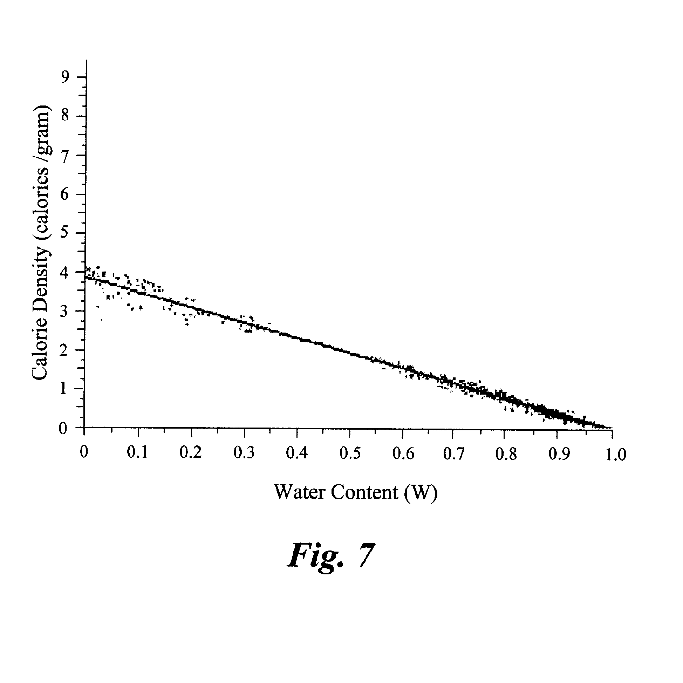

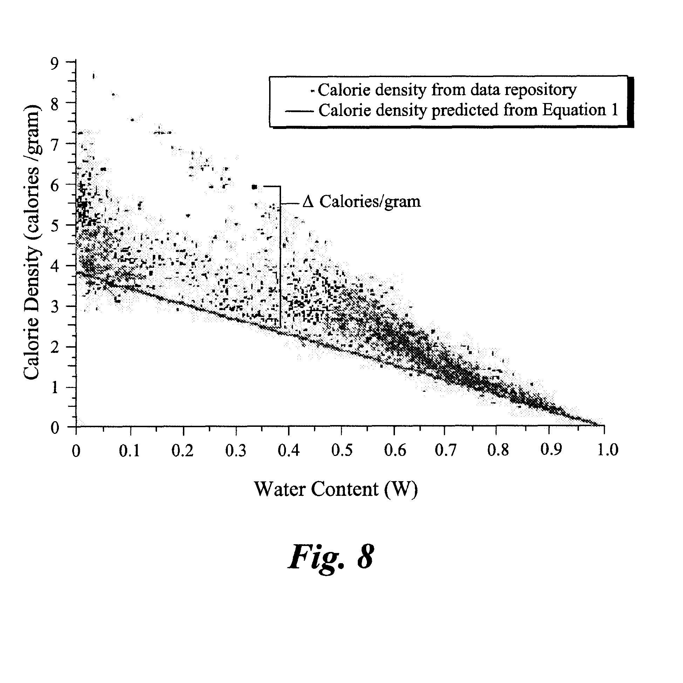

The detailed procedure 222 of generating a regression expression is described below in conjunction with FIG. 6. The regression expression can be generated by obtaining (242) water content and calorie density data associated with one or more fat-free food items. These data can be obtained, for example, by performing a series of compositional analysis tests on various fat-free food items, or from a data repository of documented nutritional information. An example of a publicly available data repository is the United States Department of Agriculture (USDA) nutritional database, which database contains water content, fat content, and calorie density data for over 6600 fat-containing and fat-free food items. The calorie density of the fat-free food items can then be plotted (244) as a function of the water content. An example of such a plot for fat-free food items reported in the USDA nutritional database is shown in FIG. 7. A linear fit to the data can be performed (246) to yield a first equation; for the data plotted in FIG.8, the first equation is found to be

$$CD = 3.79 - 3.79W \qquad \text{(Eq. 1)}$$

where W is the water content of the food sample (mass of water/total mass of the food sample) and $CD$ is the calorie density of the food sample expressed as calories/unit mass.

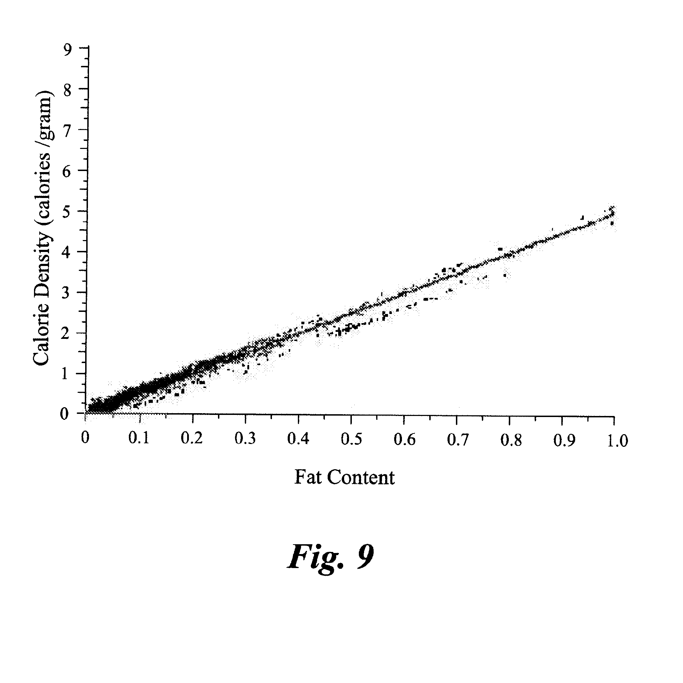

Water content, fat content, and calorie density data associated with one or more fat-containing food items can be obtained (248), again, through experimentation or from a data repository. The water content W for each of the fat-containing food items can be inputted into Equation 1 in order to calculate (250) a calorie density based solely on water content (that is, excluding the calorie density contribution of any fat contained in the food items). A plot of calorie density against water content for the fat-containing food items represented in the USDA nutritional database is provided in FIG. 8, along with a line representing the calorie density as calculated from Equation 1. The difference $\Delta CD$ between the actual calorie density (*i.e.*, the calorie density determined through separate experiments or reported in the data repository) and that calculated from Equation 1 can be determined (252); in FIG. 8, this difference is represented by the vertical distance between the actual calorie density data points and the line representing the calorie density as calculated from Equation 1. The difference $\Delta CD$ can be plotted (254) as a function of the fat content $F$ (mass of fat/total mass of the food sample) as shown in FIG. 9. A linear fit of the data can be performed (256) to yield a second equation; for the data plotted in FIG. 9, the second equation is found to be

$$\Delta CD = 5.1F \qquad \text{(Eq. 2)}$$

where, again, $\Delta CD$ is the difference between the actual calorie density of fat-containing food items reported in the USDA nutritional database and the calorie density calculated for those food items from Equation 1.

Equations 1 and 2 can be added together (258) to yield a third equation

$$CD = 3.79 - 3.79W + 5.1F \qquad \text{(Eq. 3)}$$

where, again, $CD$ is the calorie density expressed in calories/unit mass of a food sample. Equation 3 is therefore the "regression expression" that can be used to determine the calorie density of an arbitrary food sample from the fat content and the water content of the food sample. The total calorie content of a food sample is then obtained by multiplying the calculated calorie density of the food sample by the mass of the sample.

In practice, the processing unit 29 may input the water and fat content non-destructively estimated from the estimating unit 20 into the Equation 3, which equation may be pre-programmed in the processing unit 29 and/or stored in the memory 27. Additional parameters such as volume and temperature can be collected and used to calibrate the estimating unit 20 if additional accuracy is required. Empirically determined calibration functions can be stored within the processing unit 29 and/or memory 27, such that the measurement of calibration parameters and the calibration may be done automatically, without any further user input.

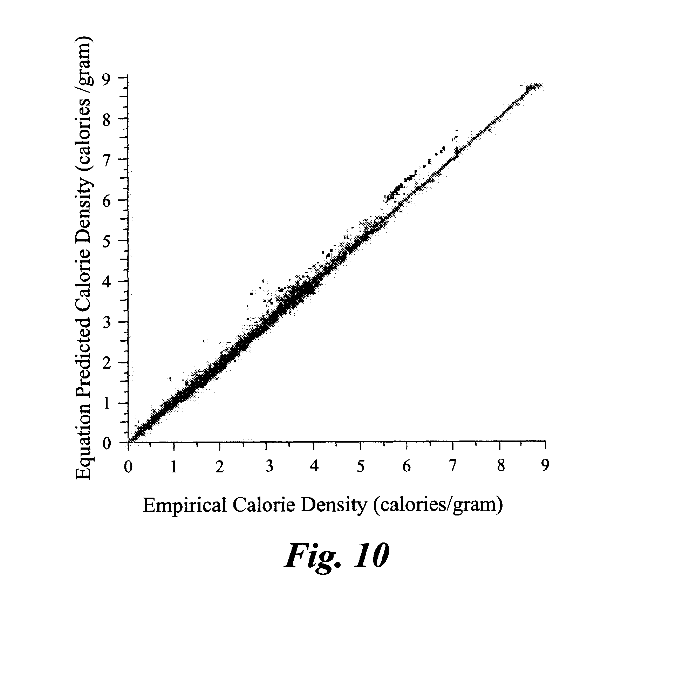

The documented calorie densities for all of the food items represented in the USDA nutritional database are plotted in FIG. 10 against the calorie densities predicted from Equation 3 for those same food items. Also shown is a line that was fitted to the data, which line has a slope of approximately unity and an $R^2$ value for the fit of 0.995. This suggests that Equation 3, which includes only fat content and water content as independent variables, may be a good predictor of calorie density. A list of some food items demonstrating the level of accuracy in using Equation 3 to predict calorie density is shown in Table 1.

Table 1.

| Food item | USDA Water Content | USDA Fat Content | USDA Calories/ gram | Equation Calories/ gram | % difference |
|---|---|---|---|---|---|
| Pizza Hut, thin and crispy, cheese | 0.388 | 0.141 | **3.04** | **3.03** | 0.28 |
| Pizza Hut, thick crust, cheese | 0.434 | 0.126 | **2.79** | **2.80** | -0.44 |
| cheesecake | 0.456 | 0.225 | **3.21** | **3.21** | -0.02 |
| fruit salad, heavy syrup | 0.766 | 0.001 | **0.89** | **0.88** | 1.36 |
| Burger King hamburger | 0.447 | 0.122 | **2.72** | **2.75** | -1.16 |
| avocado, California | 0.723 | 0.154 | **1.84** | **1.67** | 9.89 |
| egg roll | 0.513 | 0.072 | **2.21** | **2.22** | -0.32 |
| bacon | 0.125 | 0.433 | **5.52** | **5.48** | 0.81 |
| peanuts | 0.065 | 0.492 | **6.05** | **5.67** | 6.75 |
| fruit salad, water packed | 0.915 | 0.001 | **0.33** | **0.30** | 9.08 |
| onion sweet | 0.912 | 0.001 | **0.34** | **0.32** | 5.82 |
| radish, raw | 0.953 | 0.001 | **0.18** | **0.16** | 14.52 |
| sugar (high simple carbs) | 0.02 | 0 | **3.71** | **3.87** | -4.03 |
| potato | 0.4731 | 0.001 | **2.00** | **1.98** | 1.11 |
| cooked white rice | 0.6844 | 0.0028 | **1.21** | **1.30** | -6.89 |
| fried rice | 0.6099 | 0.0277 | **1.62** | **1.63** | -0.63 |
| cooked brown rice | 0.7309 | 0.009 | **1.07** | **1.11** | -3.98 |
| cooked wild rice | 0.7393 | 0.0034 | **1.01** | **1.01** | -0.46 |
| rice and beans (black) | 0.6547 | 0.0385 | **1.51** | **1.51** | -0.33 |
| cooked spaghetti noodles | 0.6213 | 0.0093 | **1.48** | **1.58** | -6.16 |
| spaghetti with meat sauce | 0.7782 | 0.0101 | **0.89** | **0.90** | -0.87 |
| Wheaties cereal | 0.0259 | 0.0333 | **3.86** | **3.67** | 5.22 |

The Applicants have therefore innovatively recognized that calorie density of an arbitrary food sample can be accurately expressed as a function of the fat and water content of that sample, without the need to collect further data related to the food sample. This is in contrast to common practices, where determination of calorie content of a food sample requires one to manually identify the calorie content of each constituent item in the food sample, for example, by researching databases of nutritional information and thereafter estimating quantities. Procedures for determining calorie density consistent with the above description may therefore be simplified as compared to conventional procedures.

Overall, systems configured in accordance with the example embodiments described above may act to estimate a calorie content of a food sample non-destructively. Estimation of the calories of the food sample may be available simply by pressing a button. As such, these systems may be well suited for integration with conventional microwave-cooking devices.

In one example embodiment, the system may be included as part of a health management module. The health management module can provide means for a user, on a real time basis, to track the calories that have been burned while simultaneously providing a means for tracking the calories in the food that the user has consumed. This system could therefore afford the user the ability to make competent and rational dietary and exercise decisions by timely comparisons of dietary and exercise activities.

While only certain features of the invention have been illustrated and described herein, many modifications and changes will occur to those skilled in the art. For example, much of the above discussion has focused on determining calorie content based on a single regression expression, such as Equation 3. However, referring to FIG. 2, in some embodiments, the memory 27 may store multiple regression expressions, with each individual regression expression being tailored, for example, to a specific class of foods. For example, the memory 27 may store a first regression expression that has been determined based on data for, say, sweets, a second regression expression that has been determined for meats, a third regression expression that has been determined for vegetables, and a fourth regression expression that has been determined from data for all of the food items in the USDA nutritional database. A user may then be given the option to invoke a food-specific regression expression where the food sample to be measured clearly falls into one of the specified categories or the general (here, the fourth) regression expression where the food sample is of unknown or nonuniform type. It is, therefore, to be understood that the appended claims are intended to cover all such modifications and changes as fall within the true spirit of the invention.

Various aspects and embodiments of the present invention are defined by the following numbered clauses:

1. A system comprising:

an estimating unit to non-destructively estimate a fat content and a water content of a food sample; and
a processing unit operatively coupled to the estimating unit to determine a calorie density based solely on the fat content and the water content of the food sample.

2. The system of clause 1, wherein the estimating unit comprises:

a spectrometer including a transmitter and a receiver; and
a weighing scale coupled to the spectrometer.

3. The system of any preceding clause, wherein the spectrometer is a microwave spectrometer, a near infrared spectrometer, or an ultra-wide band pulse dispersion microwave spectrometer.

4. The system of any preceding clause, wherein the estimating unit further comprises an optical scanner, a temperature measuring device, or a combination thereof.

5. The system of any preceding clause, wherein the optical scanner is a three-dimensional optical scanner.

6. The system of any preceding clause, wherein the temperature measuring device is an infrared thermometer.

7. The system of any preceding clause, wherein the processing unit is configured to generate a regression expression correlating a fat content and a water content and a calorie density associated with one or more food items, and wherein the fat content, the water content, and the calorie density are obtained from a data repository that is communicatively coupled to the processing unit.

8. The system of any preceding clause, wherein the processing unit is configured to calculate the calorie density of the food sample using the estimated fat content, water content and a regression expression relating fat content and water content to calorie density.

9. The system of any preceding clause, wherein the processing unit is further configured to calculate a calorie content of the food sample by multiplying the calorie density with a mass of the food sample.

10. The system of any preceding clause, further comprising:

    an activity monitoring module;
    a weight monitoring module;
    a health management module including a wireless transmitter, wherein the health management module is operatively coupled to the processing unit, the activity monitoring module, and the weight monitoring module.

11. The system of any preceding clause, wherein the activity monitoring module is configured to monitor calories burned by a user.

12. The system of any preceding clause, wherein the health management module is configured to track a weight of a user and calories consumed and burned by the user.

13. The system of any preceding clause, wherein the wireless transmiiter is configured to upload data from the processing unit, the activity monitoring module, and the weight monitoring module to the health management module.

14. The system of any preceding clause, further comprises a user interface communicatively coupled to the health management module to communicate weight, calories consumed, and calories burned to a user.

15. A method comprising:

    estimating a fat content and a water content of a food sample with an estimating unit; and
    determining a calorie density of the food sample based solely on the fat content and the water content using a processing unit, wherein the processing unit is operatively coupled to the estimating unit.

16. The method of clause 15, wherein estimating the fat content and the water content of the food sample comprises:

    weighing the food sample using a weighing scale;
    measuring a volume of the food sample using an optical scanner;
    calculating a density of the sample from the weight and the volume of the food sample; and
    estimating the fat content and the water content using a spectrometer, wherein the spectrometer is calibrated for the volume and density of the food sample.

17. The method of clause 15 or clause 16, wherein estimating the fat content and the water content of the food sample comprises:

    measuring a temperature of the food sample using a temperature-measuring device; and
    estimating the fat content and the water content using a spectrometer, wherein the spectrometer is calibrated for the temperature of the food sample.

18. The method of any of clauses 15 to 17, further compries determining a calorie content of the food sample, wherein determining the calorie content comprises:

    calculating the calorie density of the food sample by inputting the estimated fat content and the water content of the food sample in a generated regression expression; and
    calculating the calorie content of the food sample by multiplying the calorie density with a weight of the food sample.

19. The method of any of clauses 15 to 18, wherein generating the regression expression comprises:

    obtaining a water content and a calorie density associated with one or more fat free food items from a data repository;
    plotting the water content and the calorie density of the fat free food items and finding out an equation of the plot to yield a first equation;
    obtaining a water content, a fat content, and a calorie density associated with one or more fat containing food items from the data repository;
    calculating the calorie density of the fat containing food items using the first equation;
    finding out the difference between the calorie density obtained from the first equation and that reported in the data repository;

plotting the difference in calorie density and the fat content of the fat containing food items and finding out an equation of the plot to yield a second equation; and

adding the first and second equations to yield a final equation relating the calorie density and the fat content and the water content.

20. A method comprising:

transmitting microwave radiation such that at least a part of the microwave radiation interacts with a food sample;
receiving at least some of the transmitted microwave radiation;
estimating a fat content and a water content of the food sample based on the received microwave radiation and the weight of the food sample; and
determining a calorie density of the food sample based solely on the estimated fat content and the water content.

**Claims**

1. A system comprising:

   an estimating unit (21) to non-destructively estimate a fat content and a water content of a food sample; and
   a processing unit (29) operatively coupled to the estimating unit to determine a calorie density based solely on the fat content and the water content of the food sample.

2. The system of claim 1, wherein the estimating unit comprises:

   a spectrometer (70) including a transmitter (22a) and a receiver (22b); and
   a weighing scale (24) coupled to the spectrometer.

3. The system of claim 1 or claim 2, wherein the spectrometer is a microwave spectrometer, a near infrared spectrometer, or an ultra-wide band pulse dispersion microwave spectrometer.

4. The system of any preceding claim, wherein the estimating unit further comprises an optical scanner (26), a temperature measuring device (28), or a combination thereof.

5. The system of any preceding claim, wherein the processing unit is configured to generate a regression expression correlating a fat content and a water content and a calorie density associated with one or more food items, and wherein the fat content, the water content, and the calorie density are obtained from a data repository that is communicatively coupled to the processing unit.

6. The system of any preceding claim, wherein the processing unit is configured to calculate the calorie density of the food sample using the estimated fat content, water content and a regression expression relating fat content and water content to calorie density.

7. The system of any preceding claim, wherein the processing unit is further configured to calculate a calorie content of the food sample by multiplying the calorie density with a mass of the food sample.

8. The system of any preceding claim, further comprising:

   an activity monitoring module (40);
   a weight monitoring module (30);
   a health management module (10) including a wireless transmitter (12), wherein the health management module is operatively coupled to the processing unit, the activity monitoring module, and the weight monitoring module.

9. The system of any preceding claim, wherein the activity monitoring module is configured to monitor calories burned by a user.

10. The system of any preceding claim, wherein the health management module is configured to track a weight of a user and calories consumed and burned by the user.

**11.** A method comprising:

estimating a fat content and a water content of a food sample with an estimating unit; and
determining a calorie density of the food sample based solely on the fat content and the water content using a processing unit, wherein the processing unit is operatively coupled to the estimating unit.

**12.** The method of claim 11, wherein estimating the fat content and the water content of the food sample comprises:

weighing the food sample using a weighing scale;
measuring a volume of the food sample using an optical scanner;
calculating a density of the sample from the weight and the volume of the food sample; and
estimating the fat content and the water content using a spectrometer, wherein the spectrometer is calibrated for the volume and density of the food sample.

**13.** The method of claim 11 or claim 12, wherein estimating the fat content and the water content of the food sample comprises:

measuring a temperature of the food sample using a temperature-measuring device; and
estimating the fat content and the water content using a spectrometer, wherein the spectrometer is calibrated for the temperature of the food sample.

**14.** The method of any of claims 11 to 13, further compries determining a calorie content of the food sample, wherein determining the calorie content comprises:

calculating the calorie density of the food sample by inputting the estimated fat content and the water content of the food sample in a generated regression expression; and
calculating the calorie content of the food sample by multiplying the calorie density with a weight of the food sample.

**15.** The method of any of claims 11 to 14, wherein generating the regression expression comprises:

obtaining a water content and a calorie density associated with one or more fat free food items from a data repository;
plotting the water content and the calorie density of the fat free food items and finding out an equation of the plot to yield a first equation;
obtaining a water content, a fat content, and a calorie density associated with one or more fat containing food items from the data repository;
calculating the calorie density of the fat containing food items using the first equation;
finding out the difference between the calorie density obtained from the first equation and that reported in the data repository;
plotting the difference in calorie density and the fat content of the fat containing food items and finding out an equation of the plot to yield a second equation; and
adding the first and second equations to yield a final equation relating the calorie density and the fat content and the water content.

20

calorie measurement
module

30

weight-monitoring
module

10

11

12

13

40

activity-monitoring
module

50

user interface

## Fig. 1

**Fig. 2**

*200*

| Estimate fat content and water content of food sample | *202* |

↓

| Determine calorie content of food sample | *204* |

*Fig. 3*

*202*

| Measure mass of food sample | *206* |
| Measure volume of food sample | *208* |
| Measure temperature of food sample | *210* |

↓

| Transmit microwave radiation so as to interact with food sample | *214* | *212* |
| Receive transmitted microwave radiation | *216* |

↓

| Calibrate wave data based on total mass, volume, density, and temperature of food sample | *218* |

↓

| Estimate fat content and water content based on received microwave radiation and mass of food sample | *220* |

*Fig. 4*

**204**

Generate regression expression — **222**

Input values for fat and water content of food sample into regression expression — **224**

Calculate calorie density for food sample — **226**

Multiply calorie density be mass of food sample to obtain calorie content — **228**

*Fig. 5*

*222*

**242**
Obtain water content and calorie density data for fat-free foods from data repository

**244**
Plot calorie density as function of water content

**246**
Perform linear fit to calorie density-water content plot to determine Equation 1

**248**
Obtain water content and calorie density data for fat-containing foods from data repository

**250**
Calculate calorie density for food sample

**252**
Find difference ($\Delta$) between calculated calorie density and reported calorie density

**254**
Plot $\Delta$ as a function of fat content

**256**
Perform linear fit to $\Delta$-fat content plot to determine Equation 2

**258**
Sum Equation 1 and Equation 2 to obtain regression expression

*Fig. 6*

*Fig. 7*

*Fig. 8*

*Fig. 9*

*Fig. 10*

**EP 2 423 675 A2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7221169 B, Jean

- US 5331284 A, Jean

**Non-patent literature cited in the description**

- **BUFORD RANDALL JEAN.** Process Composition Monitoring at Microwave Frequencies: A Waveguide Cutoff Method and Calibration Procedure. *IEEE Transactions on Instrumentation and Measurement,* February 2006, vol. 55 (1